# EUROPEAN PATENT APPLICATION

(11) **EP 4 174 090 A1**
(43) Date of publication of application: **03.05.2023**
(21) Application number: 21849059.7
(22) Date of filing: 30.07.2021
(51) Int. Cl.: C07K 16/28, C12N 15/13, C07K 19/00, C12N 15/62, C12N 15/63, C12N 5/10, A61K 35/17, A61P 35/00

(54) **ANTI-CD133 SINGLE-CHAIN ANTIBODY AND USE THEREOF IN PREPARATION OF DRUG FOR TREATING TUMOR**

(30) Priority: 31.07.2020 CN 202010762998
(71) Applicant: Beijing Neurosurgical Institute, Beijing 100070 (CN)
(72) Inventor: ZHANG, Wei, Beijing 100070 (CN); JIANG, Tao, Beijing 100070 (CN); ZHAI, You, Beijing 100070 (CN); LI, Guanzhang, Beijing 100070 (CN)
(74) Representative: Prüfer & Partner mbB Patentanwälte · Rechtsanwälte
(86) International application number: PCT/CN2021/109863
(87) International publication number: WO 2022/022718

(57) **Abstract**

The disclosure relates to an anti-CD133 single-chain antibody. The amino acid sequence of the anti-CD133 single-chain antibody comprises a sequence shown in SEQ ID NO. 1. T lymphocytes expressing the anti-CD133 single-chain antibody can specifically kill CD133-positive tumor cells and have higher specificity and stronger killing ability.

## Description

### TECHNICAL FIELD

The disclosure relates to the field of pharmaceutical biotechnology, in particular to a chimeric antigen receptor targeting CD133, an encoding nucleic acid, an expression vector, a cell, a pharmaceutical composition and use thereof.

### BACKGROUND

Chimeric antigen receptor (CAR) is a synthetic T cell receptor consisting of an antigen binding domain, a transmembrane domain and an intracellular signaling domain. The antigen binding domain is located outside a T cell membrane, includes a single-chain antibody or ligand, and is used for specifically binding to a target antigen. The intracellular signaling domain is located in the T cell membrane, and is used for transmitting signals to a T cell to stimulate the immune response of the T cell.

The CAR can target and recognize the target antigen on the surface of a tumor cell, so the T cell expressing the CAR can be used for targeting and killing a tumor cell. However, the existing T cell expressing the CAR is still weak in killing the tumor cell.

### SUMMARY

The objective of the disclosure is to overcome the problem that the existing T cell expressing a CAR is still weak in killing a tumor cell, and to provide an anti-CD133 single-chain antibody.

In order to achieve the above objective, in a first aspect, the disclosure provides an anti-CD133 single-chain antibody, the amino acid sequence of which includes a sequence shown in SEQ ID NO. 1.

In a second aspect, the disclosure provides a nucleic acid encoding the anti-CD133 single-chain antibody according to the first aspect; and preferably, the nucleic acid has a nucleotide sequence shown in SEQ ID NO. 2.

In a third aspect, the disclosure provides a fusion protein containing an antigen binding domain, a transmembrane domain and an intracellular signaling domain that are sequentially linked; the amino acid sequence of the antigen binding domain includes the amino acid sequence of the anti-CD133 single-chain antibody according to the first aspect.

Preferably, the antigen binding domain includes the anti-CD133 single-chain antibody, and the intracellular signaling domain includes an anti-TIM3 single-chain antibody and/or IL7Rα or a truncated body of IL7Rα.

More preferably, the fusion protein has an amino acid sequence shown in any of SEQ ID NO. 3, SEQ ID NO. 4, SEQ ID NO. 5, or SEQ ID NO. 6.

In a fourth aspect, the disclosure provides a fusion nucleic acid encoding the fusion protein according to the third aspect.

Preferably, the fusion nucleic acid has a nucleotide sequence shown in any of SEQ ID NO. 7, SEQ ID NO. 8, SEQ ID NO. 9, or SEQ ID NO. 10.

In a fifth aspect, the disclosure provides an expression vector, the expression vector is inserted with an expression cassette, the expression cassette includes a first nucleic acid fragment encoding an antigen binding molecule and a second nucleic acid fragment encoding an intracellular signaling molecule, the antigen binding molecule contains the anti-CD133 single-chain antibody according to the first aspect, and an IRES element or a 2A peptide encoding sequence is inserted between the first nucleic acid fragment and the second nucleic acid fragment.

Preferably, the expression cassette is the fusion nucleic acid according to the fourth aspect.

In a sixth aspect, the disclosure provides a cell expressing a chimeric antigen receptor, the cell expressing a chimeric antigen receptor is obtained by transfection of the expression vector according to the fifth aspect by a host cell, and the chimeric antigen receptor contains the anti-CD133 single-chain antibody according to the first aspect.

Optionally, the host cell is a T cell.

In a seventh aspect, the disclosure provides use of the anti-CD133 single-chain antibody according to the first aspect, the nucleic acid according to the second aspect, the fusion protein according to the third aspect, the fusion nucleic acid according to the fourth aspect, the expression vector according to the fifth aspect, and the cell expressing a chimeric antigen receptor according to the sixth aspect in preparation of a drug for treating a tumor.

Optionally, the tumor is glioma.

In an eighth aspect, the disclosure provides a pharmaceutical composition, an active ingredient of which includes the cell expressing an anti-CD133 chimeric antigen receptor according to the sixth aspect.

According to the above technical solution, the anti-CD133 single-chain antibody provided by the disclosure can specifically recognize and bind a CD133 antigen on the surface of a tumor cell. Therefore, T lymphocytes expressing the anti-CD133 single-chain antibody can specifically kill CD133-positive tumor cells and have higher specificity and stronger killing ability.

Other features and advantages of the disclosure will be described in detail in the following specific embodiments.

### BRIEF DESCRIPTION OF THE DRAWINGS

The accompanying drawings are intended to provide a further understanding of the disclosure, constitute a part of the description, and are used for interpreting the disclosure together with the following specific embodiments, rather than limiting the disclosure. In the figures:
Fig. 1 is a flow cytometer test result diagram of CAR-T 1 cells according to an embodiment of the disclosure;
Fig. 2 is a flow cytometer test result diagram of CAR-T 2 cells according to an embodiment of the disclosure;
Fig. 3 is a flow cytometer test result diagram of CAR-T 3 cells according to an embodiment of the disclosure;
Fig. 4 is a flow cytometer test result diagram of CAR-T 4 cells according to an embodiment of the disclosure; and
Fig. 5 is a flow cytometer test result diagram of CAR-T 5 cells according to an embodiment of the disclosure.

### DETAILED DESCRIPTION

The specific embodiments of the disclosure will be described in detail below with reference to the accompanying drawings. It should be understood that the specific embodiments described herein are merely used for illustrating and interpreting the disclosure, rather than limiting the disclosure.

A first aspect of the disclosure provides an anti-CD133 single-chain antibody. The amino acid sequence of the anti-CD133 single-chain antibody includes a sequence shown in SEQ ID NO. 1.

The sequence shown in SEQ ID NO. 1 is as follows:

QVQLVQSGAEVKKPGASVKVSCKASGYTFTDFEMHWVRQAPGQGLEWMGDIDPGTG
DTAYNLKFKGRVTMTTDTSTSTAYMELRSLRSDDTAVYYCALGAFVYWGQGTLVTVSS
GSTSGGGSGGGSGGGGSSDVVMTQSPLSLPVTPGEPASISCRSSQSLANSYGNTYLSWY
LQKPGQSPQLLIYGISNRFSGVPDRFSGSGSGTDFTLKISRVEAEDVGVYYCLQGTHQPY
TFGQGTKLEIK.

CD133 is a specific marker molecule expressed independently on the surface of various tumor stem cells, so CD133 can be used as a specific target for tumor therapy. The anti-CD133 single-chain antibody was designed against CD133, and chimeric antigen receptor T cells were constructed by using the designed anti-CD133 single-chain antibody and could specifically recognize and kill CD133-positive tumor cells.

The inventors of the disclosure found that the anti-CD133 single-chain antibody can specifically recognize and bind a CD133 antigen on the surface of a tumor cell. Therefore, T lymphocytes expressing the anti-CD133 single-chain antibody can specifically kill CD133-positive tumor cells and have higher specificity and stronger killing ability.

A second aspect of the disclosure provides a nucleic acid encoding the anti-CD133 single-chain antibody according to the first aspect. Preferably, the nucleic acid has a nucleotide sequence shown in SEQ ID NO. 2.

The nucleotide sequence shown in SEQ ID NO. 2 is as follows:

caggttcagctggtgcagtctggagctgaggtgaagaagcctggggcctcagtgaaggtctcctgcaaggcttctggttac
acctttaccgactttgaaatgcactgggtgcgacaggcccctggacaagggcttgagtggatgggagatattgatcctggaactggtgata
ctgcctacaatctgaagttcaagggcagagtcaccatgaccacagacacatccacgagcacagcctacatggagctgaggagcctgag
gtctgacgacacggccgtgtattactgtgcgttgggggcctttgtttactggggccagggaaccctggtcaccgtctcctcaggcagtacta
gcggtggtggctccgggggcggttccggtgggggcggcagcagcgatgttgtgatgactcagtctccactctccctgcccgtcacccct
ggagagccggcctccatctcctgcaggtctagtcagagtcttgcaaacagttatgggaacacctatttgtcttggtacctgcagaagccagg
gcagtctccacagctcctgatctatgggatttccaacagattttctggggtccctgacaggttcagtggcagtggatcaggcacagattttac
actgaaaatcagcagagtggaggctgaggacgttggggtttattactgcttacaaggtacacatcagccgtacacgtttggccaggggac
caagctggagatcaaa.

A third aspect of the disclosure provides a fusion protein containing an antigen binding domain, a transmembrane domain and an intracellular signaling domain that are sequentially linked; the amino acid sequence of the antigen binding domain includes the amino acid sequence of the anti-CD133 single-chain antibody according to the first aspect; preferably, the antigen binding domain includes the anti-CD133 single-chain antibody, and the intracellular signaling domain includes an anti-TIM3 single-chain antibody and/or IL7Rα or a truncated body of IL7Rα; and more preferably, the fusion protein has an amino acid sequence shown in any of SEQ ID NO. 3, SEQ ID NO. 4, SEQ ID NO. 5, or SEQ ID NO. 6.

The fusion protein shown in SEQ ID NO. 3 consists of the anti-CD133 single-chain antibody, a CD28, and a CD3. The amino acid sequence shown in SEQ ID NO. 3 is as follows:

QVQLVQSGAEVKKPGASVKVSCKASGYTFTDFEMHWVRQAPGQGLEWMGD
IDPGTGDTAYNLKFKGRVTMTTDTSTSTAYMELRSLRSDDTAVYYCALGAFVYWGQGT
LVTVSSGSTSGGGSGGGSGGGGSSDVVMTQSPLSLPVTPGEPASISCRSSQSLANSYGNT
YLSWYLQKPGQSPQLLIYGISNRFSGVPDRFSGSGSGTDFTLKISRVEAEDVGVYYCLQ
GTHQPYTFGQGTKLEIKTTTPAPRPPTPAPTIASQPLSLRPEACRPAAGGAVHTRGLDFAC

DIYIWAPLAGTCGVLLLSLVITLYCRSKRSRLLHSDYMNMTPRRPGPTRKHYQPYAPPR
DFAAYRSRVKFSRSADAPAYKQGQNQLYNELNLGRREEYDVLDKRRGRDPEMGGKPR
RKNPQEGLYNELQKDKMAEAYSEIGMKGERRRGKGHDGLYQGLSTATKDTYDALHM
QALPPR.

The fusion protein shown in SEQ ID NO. 4 consists of the anti-CD133 single-chain antibody, a CD28, a truncated body of IL7Rα, and a CD3. The amino acid sequence shown in SEQ ID NO. 4 is as follows:

QVQLVQSGAEVKKPGASVKVSCKASGYTFTDFEMHWVRQAPGQGLEWMGD
IDPGTGDTAYNLKFKGRVTMTTDTSTSTAYMELRSLRSDDTAVYYCALGAFVYWGQGT
LVTVSSGSTSGGGSGGGSGGGGSSDVVMTQSPLSLPVTPGEPASISCRSSQSLANSYGNT
YLSWYLQKPGQSPQLLIYGISNRFSGVPDRFSGSGSGTDFTLKISRVEAEDVGVYYCLQ
GTHQPYTFGQGTKLEIKTTTPAPRPPTPAPTIASQPLSLRPEACRPAAGGAVHTRGLDFAC
DIYIWAPLAGTCGVLLLSLVITLYCRSKRSRLLHSDYMNMTPRRPGPTRKHYQPYAPPR
DFAAYRSKKRIKPIVWPSLPDHKKTLEHLCKKPRKNLNVSFNPESFLDCQIHRVDDIQAR
DEVEGFLQDTFPQQLEESEKQRLLGSNQEEAYVTMSSFYQNQRVKFSRSADAPAYKQG
QNQLYNELNLGRREEYDVLDKRRGRDPEMGGKPRRKNPQEGLYNELQKDKMAEAYSE
IGMKGERRRGKGHDGLYQGLSTATKDTYDALHMQALPPR.

The fusion protein shown in SEQ ID NO. 5 consists of the anti-CD133 single-chain antibody, a CD28, a CD3, a T2A, and an anti-TIM3 single-chain antibody. The amino acid sequence shown in SEQ ID NO. 5 is as follows:

QVQLVQSGAEVKKPGASVKVSCKASGYTFTDFEMHWVRQAPGQGLEWMGD
IDPGTGDTAYNLKFKGRVTMTTDTSTSTAYMELRSLRSDDTAVYYCALGAFVYWGQGT
LVTVSSGSTSGGGSGGGSGGGGSSDVVMTQSPLSLPVTPGEPASISCRSSQSLANSYGNT
YLSWYLQKPGQSPQLLIYGISNRFSGVPDRFSGSGSGTDFTLKISRVEAEDVGVYYCLQ
GTHQPYTFGQGTKLEIKTTTPAPRPPTPAPTIASQPLSLRPEACRPAAGGAVHTRGLDFAC
DIYIWAPLAGTCGVLLLSLVITLYCRSKRSRLLHSDYMNMTPRRPGPTRKHYQPYAPPR
DFAAYRSRVKFSRSADAPAYKQGQNQLYNELNLGRREEYDVLDKRRGRDPEMGGKPR
RKNPQEGLYNELQKDKMAEAYSEIGMKGERRRGKGHDGLYQGLSTATKDTYDALHM
QALPPREGRGSLLTCGDVEENPGPMTNKCLLQIALLLCFSTTALSQVQLQQSGPGLVKPS
HTLSLTCTVSGGSISSGGYYWSWTRQHPGMGLEWIGYISYSGSIYYTPSLKSRLTISVDT
SKNQFSLKLSSVTAADTAVYYCASLDSWGSNRDYWGQGTLVTVSSGGGGSEIVLTQSPA
TLSLSPGERATLSCRASQSVSSYLAWYQQKPGQAPRLLIYDASNRATGIPARFSGSGSGT
DFTLTISSLEPEDFAVYYCQQRSNWPLTFGGGTKVEIK.

The fusion protein shown in SEQ ID NO. 6 consists of the anti-CD133 single-chain antibody, a CD28, a truncated body of IL7Rα, a CD3, a T2A, and an anti-TIM3 single-chain antibody. The amino acid sequence shown in SEQ ID NO. 6 is as follows:

QVQLVQSGAEVKKPGASVKVSCKASGYTFTDFEMHWVRQAPGQGLEWMGD
IDPGTGDTAYNLKFKGRVTMTTDTSTSTAYMELRSLRSDDTAVYYCALGAFVYWGQGT
LVTVSSGSTSGGGSGGGSGGGGSSDVVMTQSPLSLPVTPGEPASISCRSSQSLANSYGNT
YLSWYLQKPGQSPQLLIYGISNRFSGVPDRFSGSGSGTDFTLKISRVEAEDVGVYYCLQ
GTHQPYTFGQGTKLEIKTTTPAPRPPTPAPTIASQPLSLRPEACRPAAGGAVHTRGLDFAC
DIYIWAPLAGTCGVLLLSLVITLYCRSKRSRLLHSDYMNMTPRRPGPTRKHYQPYAPPR
DFAAYRSKKRIKPIVWPSLPDHKKTLEHLCKKPRKNLNVSFNPESFLDCQIHRVDDIQAR
DEVEGFLQDTFPQQLEESEKQRLLGSNQEEAYVTMSSFYQNQRVKFSRSADAPAYKQG
QNQLYNELNLGRREEYDVLDKRRGRDPEMGGKPRRKNPQEGLYNELQKDKMAEAYSE
IGMKGERRRGKGHDGLYQGLSTATKDTYDALHMQALPPREGRGSLLTCGDVEENPGP
MTNKCLLQIALLLCFSTTALSQVQLQQSGPGLVKPSHTLSLTCTVSGGSISSGGYYWSW
TRQHPGMGLEWIGYISYSGSIYYTPSLKSRLTISVDTSKNQFSLKLSSVTAADTAVYYCA
SLDSWGSNRDYWGQGTLVTVSSGGGGSEIVLTQSPATLSLSPGERATLSCRASQSVSSYL
AWYQQKPGQAPRLLIYDASNRATGIPARFSGSGSGTDFTLTISSLEPEDFAVYYCQQRSN
WPLTFGGGTKVEIK.

A fourth aspect of the disclosure provides a fusion nucleic acid encoding the fusion protein according to the third aspect; and preferably, the fusion nucleic acid has a nucleotide sequence shown in any of SEQ ID NO. 7, SEQ ID NO. 8, SEQ ID NO. 9, or SEQ ID NO. 10.

The nucleotide sequence shown in SEQ ID NO. 7 is used for encoding the amino acid sequence shown in SEQ ID NO. 3. The nucleotide sequence shown in SEQ ID NO. 7 is as follows:

caggttcagctggtgcagtctggagctgaggtgaagaagcctggggcctcagtgaaggtctcctgcaaggcttctggttac
acctttaccgactttgaaatgcactgggtgcgacaggcccctggacaagggcttgagtggatgggagatattgatcctggaactggtgata
ctgcctacaatctgaagttcaagggcagagtcaccatgaccacagacacatccacgagcacagcctacatggagctgaggagcctgag
gtctgacgacacggccgtgtattactgtgcgttgggggcctttgtttactggggccagggaaccctggtcaccgtctcctcaggcagtacta
gcggtggtggctccgggggcggttccggtgggggcggcagcagcgatgttgtgatgactcagtctccactctccctgcccgtcacccct
ggagagccggcctccatctcctgcaggtctagtcagagtcttgcaaacagttatgggaacacctatttgtcttggtacctgcagaagccagg
gcagtctccacagctcctgatctatgggatttccaacagattttctggggtccctgacaggttcagtggcagtggatcaggcacagattttac
actgaaaatcagcagagtggaggctgaggacgttggggtttattactgcttacaaggtacacatcagccgtacacgtttggccaggggac
caagctggagatcaaaaccactaccccagcaccgaggccacccaccccggctcctaccatcgcctcccagcctctgtccctgcgtccgg
aggcatgtagacccgcagctggtggggccgtgcatacccggggtcttgacttcgcctgcgatatctacatttgggcccctctggctggtac
ttgcggggtcctgctgctttcactcgtgatcactctttactgtaggagtaagaggagcaggctcctgcacagtgactacatgaacatgactcc
ccgccgccccgggcccacccgcaagcattaccagccctatgccccaccacgcgacttcgcagcctatcgctcccgcgtgaaattcagcc
gcagcgcagatgctccagcctacaagcaggggcagaaccagctctacaacgaactcaatcttggtcggagagaggagtacgacgtgct

ggacaagcggagaggacgggacccagaaatgggcgggaagccgcgcagaaagaatccccaagagggcctgtacaacgagctccaa
aaggataagatggcagaagcctatagcgagattggtatgaaaggggaacgcagaagaggcaaaggccacgacggactgtaccaggg
actcagcaccgccaccaaggacacctatgacgctcttcacatgcaggccctgccgcctcgg.

The nucleotide sequence shown in SEQ ID NO. 8 is used for encoding the amino acid sequence shown in SEQ ID NO. 4. The nucleotide sequence shown in SEQ ID NO. 8 is as follows:

caggttcagctggtgcagtctggagctgaggtgaagaagcctggggcctcagtgaaggtctcctgcaaggcttctggttac
acctttaccgactttgaaatgcactgggtgcgacaggcccctggacaagggcttgagtggatgggagatattgatcctggaactggtgata
ctgcctacaatctgaagttcaagggcagagtcaccatgaccacagacacatccacgagcacagcctacatggagctgaggagcctgag
gtctgacgacacggccgtgtattactgtgcgttgggggcctttgtttactggggccagggaaccctggtcaccgtctcctcaggcagtacta
gcggtggtggctccgggggcggttccggtgggggcggcagcagcgatgttgtgatgactcagtctccactctccctgcccgtcacccct
ggagagccggcctccatctcctgcaggtctagtcagagtcttgcaaacagttatgggaacacctatttgtcttggtacctgcagaagccagg
gcagtctccacagctcctgatctatgggatttccaacagattttctggggtccctgacaggttcagtggcagtggatcaggcacagattttac
actgaaaatcagcagagtggaggctgaggacgttggggtttattactgcttacaaggtacacatcagccgtacacgtttggccaggggac
caagctggagatcaaaaccactaccccagcaccgaggccacccaccccggctcctaccatcgcctcccagcctctgtccctgcgtccgg
aggcatgtagacccgcagctggtggggccgtgcatacccggggtcttgacttcgcctgcgatatctacatttgggcccctctggctggtac
ttgcggggtcctgctgctttcactcgtgatcactctttactgtaggagtaagaggagcaggctcctgcacagtgactacatgaacatgactcc
ccgccgccccgggcccacccgcaagcattaccagccctatgccccaccacgcgacttcgcagcctatcgctccaaaaaaaggattaag
cctatcgtatggcccagtctccccgatcacaagaagactctggaacacctttgtaagaaaccaagaaaaaatttaaatgtgagtttcaatcct
gaaagtttcctggactgccagattcatagggtggatgacattcaagctagagatgaagtggaaggttttctgcaagatacgtttcctcagcaa
ctagaagaatctgagaagcagaggcttctgggatcaaatcaagaagaagcatatgtcaccatgtccagcttctaccaaaaccagcgcgtg
aaattcagccgcagcgcagatgctccagcctacaagcaggggcagaaccagctctacaacgaactcaatcttggtcggagagaggagt
acgacgtgctggacaagcggagaggacgggacccagaaatgggcgggaagccgcgcagaaagaatccccaagagggcctgtacaa
cgagctccaaaaggataagatggcagaagcctatagcgagattggtatgaaaggggaacgcagaagaggcaaaggccacgacggact
gtaccagggactcagcaccgccaccaaggacacctatgacgctcttcacatgcaggccctgccgcctcgg.

The nucleotide sequence shown in SEQ ID NO. 9 is used for encoding the amino acid sequence shown in SEQ ID NO. 5. The nucleotide sequence shown in SEQ ID NO. 9 is as follows:

caggttcagctggtgcagtctggagctgaggtgaagaagcctggggcctcagtgaaggtctcctgcaaggcttctggttac
acctttaccgactttgaaatgcactgggtgcgacaggcccctggacaagggcttgagtggatgggagatattgatcctggaactggtgata
ctgcctacaatctgaagttcaagggcagagtcaccatgaccacagacacatccacgagcacagcctacatggagctgaggagcctgag
gtctgacgacacggccgtgtattactgtgcgttgggggcctttgtttactggggccagggaaccctggtcaccgtctcctcaggcagtacta
gcggtggtggctccgggggcggttccggtgggggcggcagcagcgatgttgtgatgactcagtctccactctccctgcccgtcacccct
ggagagccggcctccatctcctgcaggtctagtcagagtcttgcaaacagttatgggaacacctatttgtcttggtacctgcagaagccagg
gcagtctccacagctcctgatctatgggatttccaacagattttctggggtccctgacaggttcagtggcagtggatcaggcacagattttac

actgaaaatcagcagagtggaggctgaggacgttggggtttattactgcttacaaggtacacatcagccgtacacgtttggccaggggac
caagctggagatcaaaaccactaccccagcaccgaggccacccaccccggctcctaccatcgcctcccagcctctgtccctgcgtccgg
aggcatgtagacccgcagctggtggggccgtgcatacccggggtcttgacttcgcctgcgatatctacatttgggcccctctggctggtac
ttgcggggtcctgctgctttcactcgtgatcactctttactgtaggagtaagaggagcaggctcctgcacagtgactacatgaacatgactcc
ccgccgccccgggcccacccgcaagcattaccagccctatgccccaccacgcgacttcgcagcctatcgctcccgcgtgaaattcagcc
gcagcgcagatgctccagcctacaagcaggggcagaaccagctctacaacgaactcaatcttggtcggagagaggagtacgacgtgct
ggacaagcggagaggacgggacccagaaatgggcgggaagccgcgcagaaagaatccccaagagggcctgtacaacgagctccaa
aaggataagatggcagaagcctatagcgagattggtatgaaaggggaacgcagaagaggcaaaggccacgacggactgtaccaggg
actcagcaccgccaccaaggacacctatgacgctcttcacatgcaggccctgccgcctcgggagggaaggggttctctcctgacctgcg
gcgatgtggaggagaaccccggacccatgaccaacaagtgtctcctccaaattgctctcctgttgtgcttctccactacagctctttcccagg
tgcagctacagcagtcgggcccaggactggtgaagccttcacacaccctgtccctcacctgcactgtctctggtggctccatcagcagtgg
tggttactactggagttggacccgtcagcacccagggatgggcctggagtggattggatacatctcttacagtgggagtatctattacactc
cgtccctcaagagtcgacttaccatatcagtggacacgtctaagaaccagttctccctgaagctgagctctgtgactgccgcggacacggc
cgtatattactgtgcgagtttggattcctggggatctaaccgtgactactggggccagggaaccctggtcaccgtctcgagtggaggtggt
ggatccgaaattgtgttgacgcagtctccagccaccctgtctttgtctccaggggaaagagccaccctctcctgcagggccagtcagagtg
ttagcagctacttagcctggtaccaacagaaacctggccaggctcccaggctcctcatctatgatgcatccaacagggccactggcatccc
agccaggttcagtggcagtgggtctgggacagacttcactctcaccatcagcagcctagagcctgaagattttgcagtttattactgtcagc
agcgtagcaactggccgctcactttcggcggagggaccaaggtggagattaag.

The nucleotide sequence shown in SEQ ID NO. 10 is used for encoding the amino acid sequence shown in SEQ ID NO. 6. The nucleotide sequence shown in SEQ ID NO. 10 is as follows:

caggttcagctggtgcagtctggagctgaggtgaagaagcctggggcctcagtgaaggtctcctgcaaggcttctggttac
acctttaccgactttgaaatgcactgggtgcgacaggcccctggacaagggcttgagtggatgggagatattgatcctggaactggtgata
ctgcctacaatctgaagttcaagggcagagtcaccatgaccacagacacatccacgagcacagcctacatggagctgaggagcctgag
gtctgacgacacggccgtgtattactgtgcgttgggggcctttgtttactggggccagggaaccctggtcaccgtctcctcaggcagtacta
gcggtggtggctccgggggcggttccggtgggggcggcagcagcgatgttgtgatgactcagtctccactctccctgcccgtcacccct
ggagagccggcctccatctcctgcaggtctagtcagagtcttgcaaacagttatgggaacacctatttgtcttggtacctgcagaagccagg
gcagtctccacagctcctgatctatgggatttccaacagattttctggggtccctgacaggttcagtggcagtggatcaggcacagattttac
actgaaaatcagcagagtggaggctgaggacgttggggtttattactgcttacaaggtacacatcagccgtacacgtttggccaggggac
caagctggagatcaaaaccactaccccagcaccgaggccacccaccccggctcctaccatcgcctcccagcctctgtccctgcgtccgg
aggcatgtagacccgcagctggtggggccgtgcatacccggggtcttgacttcgcctgcgatatctacatttgggcccctctggctggtac
ttgcggggtcctgctgctttcactcgtgatcactctttactgtaggagtaagaggagcaggctcctgcacagtgactacatgaacatgactcc
ccgccgccccgggcccacccgcaagcattaccagccctatgccccaccacgcgacttcgcagcctatcgctccaaaaaaaggattaag
cctatcgtatggcccagtctccccgatcacaagaagactctggaacacctttgtaagaaaccaagaaaaaatttaaatgtgagtttcaatcct
gaaagtttcctggactgccagattcatagggtggatgacattcaagctagagatgaagtggaaggttttctgcaagatacgtttcctcagcaa

ctagaagaatctgagaagcagaggcttctgggatcaaatcaagaagaagcatatgtcaccatgtccagcttctaccaaaaccagcgcgtg
aaattcagccgcagcgcagatgctccagcctacaagcaggggcagaaccagctctacaacgaactcaatcttggtcggagagaggagt
acgacgtgctggacaagcggagaggacgggacccagaaatgggcgggaagccgcgcagaaagaatccccaagagggcctgtacaa
cgagctccaaaaggataagatggcagaagcctatagcgagattggtatgaaaggggaacgcagaagaggcaaaggccacgacggact
gtaccagggactcagcaccgccaccaaggacacctatgacgctcttcacatgcaggccctgccgcctcgggagggaaggggttctctcc
tgacctgcggcgatgtggaggagaaccccggacccatgaccaacaagtgtctcctccaaattgctctcctgttgtgcttctccactacagct
ctttcccaggtgcagctacagcagtcgggcccaggactggtgaagccttcacacaccctgtccctcacctgcactgtctctggtggctccat
cagcagtggtggttactactggagttggacccgtcagcacccagggatgggcctggagtggattggatacatctcttacagtgggagtatc
tattacactccgtccctcaagagtcgacttaccatatcagtggacacgtctaagaaccagttctccctgaagctgagctctgtgactgccgcg
gacacggccgtatattactgtgcgagtttggattcctggggatctaaccgtgactactggggccagggaaccctggtcaccgtctcgagtg
gaggtggtggatccgaaattgtgttgacgcagtctccagccaccctgtctttgtctccaggggaaagagccaccctctcctgcagggccag
tcagagtgttagcagctacttagcctggtaccaacagaaacctggccaggctcccaggctcctcatctatgatgcatccaacagggccact
ggcatcccagccaggttcagtggcagtgggtctgggacagacttcactctcaccatcagcagcctagagcctgaagattttgcagtttatta
ctgtcagcagcgtagcaactggccgctcactttcggcggagggaccaaggtggagattaagcgctcactttcggcggagggaccaaggt
ggagattaag.

A fifth aspect of the disclosure provides an expression vector, the expression vector is inserted with an expression cassette, the expression cassette includes a first nucleic acid fragment encoding an antigen binding molecule and a second nucleic acid fragment encoding an intracellular signaling molecule, the antigen binding molecule contains the anti-CD133 single-chain antibody according to the first aspect, and an IRES element or a 2A peptide encoding sequence is inserted between the first nucleic acid fragment and the second nucleic acid fragment; and preferably, the expression cassette is the fusion nucleic acid according to the fourth aspect.

A sixth aspect of the disclosure provides a cell expressing a chimeric antigen receptor, the cell expressing a chimeric antigen receptor is obtained by transfection of the expression vector according to the fifth aspect by a host cell, and the chimeric antigen receptor contains the anti-CD133 single-chain antibody according to the first aspect. Optionally, the host cell is a T cell.

A seventh aspect of the disclosure provides use of the anti-CD133 single-chain antibody according to the first aspect, the nucleic acid according to the second aspect, the fusion protein according to the third aspect, the fusion nucleic acid according to the fourth aspect, the expression vector according to the fifth aspect, and the cell expressing a chimeric antigen receptor according to the sixth aspect in preparation of a drug for treating a tumor. Optionally, the tumor is glioma.

An eighth aspect of the disclosure provides a pharmaceutical composition, an active ingredient of which includes the cell expressing an anti-CD133 chimeric antigen receptor according to the sixth aspect.

The disclosure is further described by the following examples, but the disclosure is not limited thereby.

### Example 1

This example was used to explain the construction of expression vectors.
(1) Nucleotide sequences shown in SEQ ID NO. 7 to 10 were respectively synthesized by using a full sequence synthesis method. The nucleotide sequences shown in SEQ ID NO. 7 to 10 were used for encoding fusion proteins shown in SEQ ID NO. 3 to 6. The compositions of the fusion proteins shown in SEQ ID NO. 3 to 6 were as follows:
   G1: the fusion protein shown in SEQ ID NO. 3 consisted of an anti-CD133 single-chain antibody, a CD28, and a CD3;
   G2: the fusion protein shown in SEQ ID NO. 4 consisted of an anti-CD133 single-chain antibody, a CD28, a truncated body of IL7Rα, and a CD3;
   G3: the fusion protein shown in SEQ ID NO. 5 consisted of an anti-CD133 single-chain antibody, a CD28, a CD3, a T2A, and an anti-TIM3 single-chain antibody;
   G4: the fusion protein shown in SEQ ID NO. 6 consisted of an anti-CD133 single-chain antibody, a CD28, a truncated body of IL7Rα, a CD3, a T2A, and an anti-TIM3 single-chain antibody.
(2) The four kinds of nucleotide sequences synthesized in step (1) were respectively inserted into pLVX-IRES-ΔNGFR (purchased from Clontech Company, item number 631982) as a vector to obtain four kinds of lentivirus expression vectors of this example.

### Example 2

This example was used to explain the preparation and test of T cells expressing an anti-CD133 single-chain antibody.
(1) The four kinds of lentivirus expression vectors constructed in Example 1 and an empty pLVX-IRES-ΔNGFR vector were packaged with lentiviruses respectively, and then T cells were cultured in vitro, transfected and proliferated according to the following methods.
(2) T cells in blood were separated according to the following method: 1 mL of sterile PBS and 1 mL of blood were mixed evenly, then slowly added to an upper layer of a lymphocyte separation medium Ficoll, and centrifuged at 4°C and 400 g for 30 min, with acceleration and deceleration set to 0 respectively. After centrifugation, the upper plasma was removed, the middle white membrane cells were pipetted, PBS was added for re-suspension washing, and centrifugation was carried out for 10 min at 100 g, with normal acceleration and deceleration. After centrifugation, the upper washing solution was removed, 1mL of 1640 + 10% FBS + 1% double antibody + 1 × Glutamine medium was added to re-suspend cells, and then the cells were stimulated to proliferate by means of anti-human CD3/CD28 magnetic beads (purchased from Thermo Fisher company), where the concentration of re-suspended cells was 1 × 10⁶ cells/mL and the amount of magnetic beads added was 100 µL. Then 100 IU/mL rhIL-2 (Peprotech) was added to stimulate culture for 2 days to obtain T cells.
(3) Transfection of lentiviruses was carried out according to the following method: the lentiviruses packaged in step (1) were respectively added to five parts of T cells separated above, and then polybrene with a final concentration of 6 µg/mL was added, followed by uniform mixing and centrifugation at 32°C and 800 g for 100 min. After centrifugation, the culture was continued in an incubator for 24 h. After the culture, the culture solution was centrifuged at 1500 rpm for 15 min, and the centrifuged cells were inoculated at a density of 1×10⁶/mL into a culture plate and stimulated for culture with rhIL2-100 IU/mL. After that, the medium was changed every 2-3 days until 2-4 weeks, and transfected T lymphocytes CAR-T 1, CAR-T 2, CAR-T 3, CAR-T 4, and CAR-T 5 were obtained. CAR-T 1 was transfected with the nucleotide sequence shown in SEQ ID NO. 7 and could express the fusion protein shown in SEQ ID NO. 3; CAR-T 2 was transfected with the nucleotide sequence shown in SEQ ID NO. 8 and could express the fusion protein shown in SEQ ID NO. 4; CAR-T 3 was transfected with the nucleotide sequence shown in SEQ ID NO. 9 and could express the fusion protein shown in SEQ ID NO. 5; CAR-T 4 was transfected with the nucleotide sequence shown in SEQ ID NO. 10 and could express the fusion protein shown in SEQ ID NO. 6; and CAR-T 5 was transfected with the empty vector.

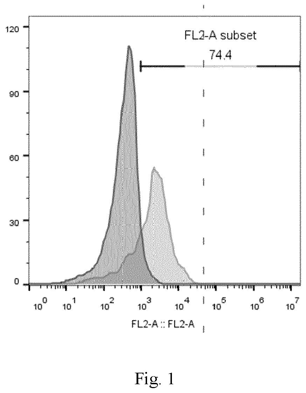

After culture, the cells were re-suspended with PBS, and the ratio of the above five kinds of transfected T lymphocytes and the expression of CAR protein on the surface were tested with a flow cytometer. The test method was as follows: the T cells to be tested after transfection were centrifuged and collected respectively, the supernatant was discarded after the T cells to be tested were washed with PBS once, and a corresponding test amount of monoclonal antibody was added according to antibody instructions and kept away from light for 30 min, followed by PBS washing, re-suspension, filtration with a membrane, and test with a flow cytometer, where the antibody used for the test was a PE labeled goat anti-human VH+VL flow antibody. The results were shown in Figs. 1-5.
Fig. 1 is a flow cytometer test result diagram of CAR-T 1 cells provided by an embodiment of the disclosure;

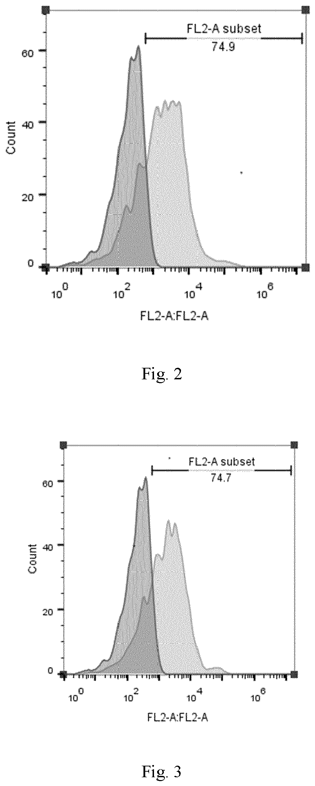

Fig. 2 is a flow cytometer test result diagram of CAR-T 2 cells provided by an embodiment of the disclosure;
Fig. 3 is a flow cytometer test result diagram of CAR-T 3 cells provided by an embodiment of the disclosure;

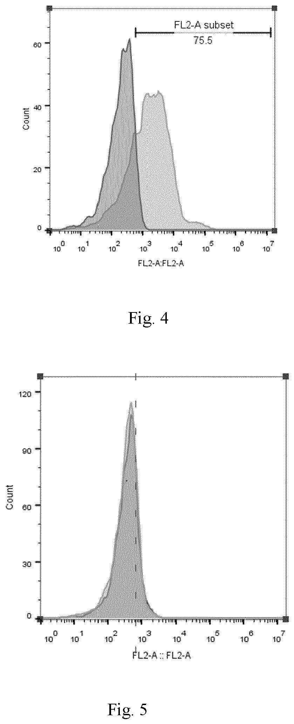

Fig. 4 is a flow cytometer test result diagram of CAR-T 4 cells provided by an embodiment of the disclosure; and
Fig. 5 is a flow cytometer test result diagram of CAR-T 5 cells according to an embodiment of the disclosure.

As can be seen from Figs. 1-4, other cells different from normal T lymphocytes were detected out from the CAR-T 1 cells, the CAR-T 2 cells, the CAR-T 3 cells, and the CAR-T 4 cells. As can be seen from Fig. 5, other cells different from normal T lymphocytes were not detected out from the CAR-T 5 cells. This showed that the CAR-T cells transfected with fusion genes in this example had successfully expressed the target fusion protein.

### Example 3

This example was used to verify the ability of T cells expressing an anti-CD133 single-chain antibody to kill CD133-positive tumor cells.

Kits for LDH release tests in this example were purchased from DOJINDO.

The five kinds of CAR-T cells obtained by transfection and culture in Example 2 were respectively mixed with CD44- and CD133-positive glioma stem cells GSC20 according to different effect-target ratios (the number of T cells: the number of glioma stem cells). The mixed cells were cultured in 96-well plates, where each well contained 4 × 10⁴ glioma stem cells, and the reaction system was 200 µL per well. The culture conditions included: 37°C, 5% CO₂, and culture in a saturated humidity incubator for 4 h.

Determination of lactate dehydrogenase activity: after centrifugation, 100 µL of supernatant was pipetted from each well and placed in 96-well enzyme-labeled plates, 100 µL of LDH substrate was added to each well, and the reaction was carried out at room temperature for 30 min without light. After the reaction, 50 µL of termination solution was added to each well to terminate the enzymatic reaction. The optical density (OD) was measured with a microplate reader 490 nm. The average optical density (OD) of each group was calculated, and the lysis rate of glioma stem cells by each kind of T cells was calculated according to the following formula. The results were shown in Table 1.

Lysis rate% = (OD of the experimental group - OD spontaneously released by glioma stem cells - OD naturally released by effector cells)/ (maximum OD released by glioma stem cells - OD spontaneously released by glioma stem cells)

**Table 1**

| CAR-T cells | Lysis rate of glioma stem cells by CAR-T cells under different effect-target ratios (%) | | | |
|---|---|---|---|---|
| | 5:1 | 3:1 | 2:1 | 1:1 |
| CAR-T 1 | 41.36% | 33.52% | 25.99% | 6.53% |
| CAR-T 2 | 43.35% | 39.65% | 28.01% | 16.30% |
| CAR-T 3 | 49.47% | 35.26% | 20.34% | 16.91% |
| CAR-T 4 | 87.24% | 69.58% | 36.79% | 31.41% |
| CAR-T 5 | 6.87% | 2.63% | 1.23% | 2.41% |

As can be seen from Table 1, T lymphocytes expressing the anti-CD133 single-chain antibody provided by the disclosure can specifically kill CD133-positive tumor cells and have higher specificity and stronger killing ability.

### Comparative Example

Conventional second generation CAR-T cells (EGFR vIII-CD28-CD3) targeting a classical tumor target EGFR vIII were used to replace the T cells in Example 3, and then killing rates of glioma stem cells GSC20 by CAR-T under different effect-target ratios were tested according to the method of Example 3. The test result is shown in Table 2.

**Table 2**

| | Lysis rate of glioma stem cells by conventional second generation CAR-T cells under different effect-target ratios (%) | | | |
|---|---|---|---|---|
| Effect-target ratio | 5:1 | 3:1 | 2:1 | 1:1 |
| Conventional CAR-T | 17.57% | 14.62% | 13.97% | 11.88% |

As can be seen from Table 2, the ability of the conventional second generation CAR-T cells targeting a classical tumor stem cell target EGFR vIII to kill glioma stem cells was weaker than that of the T cells expressing the anti-CD133 single-chain antibody provided by the disclosure.

The preferred embodiments of the disclosure are described in detail above with reference to the accompanying drawings. However, the disclosure is not limited to the specific details in the above embodiments. Various simple variations may be made to the technical solutions of the disclosure within the scope of the technical idea of the disclosure, and these simple variations fall within the scope of the disclosure.

It should be further noted that the specific technical features described in the above specific embodiments may be combined in any suitable manner without contradiction. In order to avoid unnecessary repetition, the disclosure will not describe various possible combinations.

Moreover, the various different embodiments of the disclosure may be combined randomly without deviating from the idea of the disclosure, and the combinations should also be regarded as the disclosure of the disclosure.

## Claims

1. An anti-CD133 single-chain antibody, wherein the amino acid sequence of the anti-CD133 single-chain antibody comprises a sequence shown in SEQ ID NO. 1.

2. A nucleic acid, wherein the nucleic acid encodes the anti-CD133 single-chain antibody according to claim 1; and
preferably, the nucleic acid has a nucleotide sequence shown in SEQ ID NO. 2.

3. A fusion protein, wherein the fusion protein contains an antigen binding domain, a transmembrane domain and an intracellular signaling domain that are sequentially linked; the amino acid sequence of the antigen binding domain comprises the amino acid sequence of the anti-CD133 single-chain antibody according to claim 1;
preferably, the antigen binding domain comprises the anti-CD133 single-chain antibody, and the intracellular signaling domain comprises an anti-TIM3 single-chain antibody and/or IL7Rα or a truncated body of IL7Rα; and
more preferably, the fusion protein has an amino acid sequence shown in any of SEQ ID NO. 3, SEQ ID NO. 4, SEQ ID NO. 5, or SEQ ID NO. 6.

4. A fusion nucleic acid, wherein the fusion nucleic acid encodes the fusion protein according to claim 3; and
preferably, the fusion nucleic acid has a nucleotide sequence shown in any of SEQ ID NO. 7, SEQ ID NO. 8, SEQ ID NO. 9, or SEQ ID NO. 10.

5. An expression vector, wherein the expression vector is inserted with an expression cassette, the expression cassette comprises a first nucleic acid fragment encoding an antigen binding molecule and a second nucleic acid fragment encoding an intracellular signaling molecule, the antigen binding molecule contains the anti-CD133 single-chain antibody according to claim 1, and an IRES element or a 2A peptide encoding sequence is inserted between the first nucleic acid fragment and the second nucleic acid fragment; and
preferably, the expression cassette is the fusion nucleic acid according to claim 4.

6. A cell expressing a chimeric antigen receptor, wherein the cell expressing a chimeric antigen receptor is obtained by transfection of the expression vector according to claim 5 by a host cell, and the chimeric antigen receptor contains the anti-CD133 single-chain antibody according to claim 1.

7. The cell according to claim 6, wherein the host cell is a T cell.

8. Use of the anti-CD133 single-chain antibody according to claim 1, the nucleic acid according to claim 2, the fusion protein according to claim 3, the fusion nucleic acid according to claim 4, the expression vector according to claim 5, and the cell expressing a chimeric antigen receptor according to claim 6 or 7 in preparation of a drug for treating a tumor.

9. The use according to claim 8, wherein the tumor is a glioma.

10. A pharmaceutical composition, wherein an active ingredient of the pharmaceutical composition comprises the cell expressing an anti-CD133 chimeric antigen receptor according to claim 6 or 7.
